# EUROPEAN PATENT SPECIFICATION

(11) **EP 3 738 449 B1**
(45) Date of publication and mention of the grant of the patent: **08.11.2023**
(21) Application number: 20172210.5
(22) Date of filing: 29.04.2020
(51) Int. Cl.: A24F 40/10, A24F 40/51

(54) **ELECTRONIC ATOMIZING APPARATUS, CONTROL METHOD THEREOF, ATOMIZER AND POWER SUPPLY ASSEMBLY**
ELEKTRONISCHE ZERSTÄUBUNGSVORRICHTUNG, STEUERUNGSVERFAHREN DAFÜR, ZERSTÄUBER UND STROMVERSORGUNGSANORDNUNG
APPAREIL D'ATOMISATION ÉLECTRONIQUE, PROCÉDÉ DE COMMANDE ASSOCIÉ, ATOMISEUR ET ENSEMBLE D'ALIMENTATION ÉLECTRIQUE

(30) Priority: 16.05.2019 CN 201910409910
(43) Date of publication of application: 18.11.2020
(73) Proprietor: Shenzhen Smoore Technology Limited, Shenzhen, Guangdong 518102 (CN)
(72) Inventor: ZHOU, Jun, Shenzhen, Guangdong 518102 (CN)
(74) Representative: de Arpe Fernandez, Manuel

(56) References cited:
- EP-A2- 2 875 740
- WO-A2-2013/138384
- CN-A- 109 426 171
- US-A1- 2015 305 409
- US-A1- 2016 158 782

## Description

### TECHNICAL FIELD

The present disclosure relates to the field of daily electronic products, and more particular to an electronic atomizing apparatus and a control method thereof.

### BACKGROUND

An electronic atomizing apparatus, as an alternative to a cigarette article, is mostly used for smoking cessation. The electronic atomizing apparatus has a similar appearance and taste to a cigarette, but does not contain harmful components such as tar and suspended particles generally found in conventional cigarettes, and thus could reduce harm to the human body.

The inventors of the present disclosure have found in long-term research and development that the liquid storage chamber in the electronic atomizing apparatus is generally arranged between the mouthpiece and the heater. If the user does not properly operate the electronic atomizing apparatus during use, potential safety hazard is resulted. When the electronic atomizing apparatus is inverted, there is little or no aerosol-generating liquid heated by the heater, resulting in the heater being in a dry burning state, thereby damaging the electronic atomizing apparatus. Further in the dry burning state, harmful substances is produced and inhaled by the user with the smoke, which could be harmful to the health of the user.

American Patent Application No. US2016/0158782 discloses an aerosol delivery device. The aerosol delivery device includes a housing, motion sensor and microprocessor. The motion sensor is within the housing and configured to detect a defined motion of the aerosol delivery device caused by user interaction with the housing to perform a gesture. The motion sensor is configured to convert the defined motion to an electrical signal. The microprocessor or motion sensor, then, is configured to receive the electrical signal, recognize the gesture and an operation associated with the gesture based on the electrical signal, and control at least one functional element of the aerosol delivery device to perform the operation.

Chinese Patent Application No. CN109426171 provides an electronic cigarette and a control method thereof. The method comprises that after the electronic cigarette is switched on, a magnetic induction circuit starts working; when the liquid level of cigarette liquid stored in a liquid storage cavity of the electronic cigarette is higher than or equivalent to the predetermined liquid level, a floating body in the liquid storage cavity floats to a first position, in the first position, a magnetic induction switch assembly is conducted under effect of a magnet in the floating body, and the electronic cigarette controls an atomizing assembly to work when receiving a smoking signal; and when the liquid level of cigarette liquid stored in the liquid storage cavity of the electronic cigarette is lower than the predetermined liquid level, the height of the floating body in the liquid storage cavity is lower than a first height, the magnetic induction switch assembly is disconnected, and prompt information is emitted. Thus, the electronic cigarette is prevented from dry burning when there is too little cigarette liquid or completely no cigarette liquid, and the user and equipment safety is protected.

American Patent Application No. US 2015/0305409 provides an electronic cigarette or vaporizer. The electronic cigarette or vaporizer includes a shell and a cartomizer receivable within a chamber within a portion of the shell. The cartomizer holds a vaporizable fluid, dry substance, or other vaporizable substance such as a wax. A charger is also included, which in one embodiment is insertable into the chamber, and in another embodiment is connectable to an end of the shell. Various electrical components, such as a printed circuit board, a rechargeable battery, various indicator lights, a sensor, and display screen, for instance, improve the functionality of the vaporizer from known vaporizers. Magnets is also provided in order to secure the cartomizer or the charger to the shell.

The International Application Number PCT/US20 13/030610 discloses devices and methods for vaporizing active ingredients of a selected substance for inhalation using a portable vaporization device. In certain aspects, the device includes a portable power source, a heating portion, an inhalation sensor, a temperature sensor, a distal light source, and a grinding portion. In response to an inhalation by a user, the power source energizes a heating element of the heating portion so as to heat air flow to a desired vaporization temperature within a few seconds of detecting inhalation, using convection and radiative heating. The device includes a receptacle for receiving a cartridge containing a pre-prepared substance, such as a liquid, gel, powder, or solid brick, and a grinding portion to allow a user to grind intact portions of cellulose-based material into smaller pieces to facilitate vaporization by manually rotating portions of the device relative to each other.

European Patent Application No. EP2875740 discloses an electronic cigarette (10). The electronic cigarette (10) includes a shell (106) and a cartomizer (200) receivable within a chamber (108) within a portion of the shell. The cartomizer holds a vaporizable fluid (300), dry substance, or other vaporizable substance such as a wax. A charger is also included which in one embodiment is insertable into the chamber, and in another embodiment is connectable to an end of the shell. Various electrical components, such as a printed circuit board (112), a rechargeable battery (110), various indicator lights (114,116), a sensor, and display screen, for instance, improve the functionality of the vaporizer from known vaporizers. Magnets (202) are also provided in order to secure the cartomizer or the charger to the shell.

### SUMMARY

According to an aspect of the present disclosure, an electronic atomizing apparatus is provided. The electronic atomizing apparatus according to the invention is as defined in any of claims 1-10.

According to another aspect of the present disclosure, a control method of an electronic atomizing apparatus is provided. The control method according to the invention is as defined in any of claims 11-13.

In the present disclosure, an angle detection mechanism is arranged in the electronic atomizing apparatus to detect the tilt angle of the electronic atomizing apparatus. When it is determined that the tilt angle is less than the angle threshold, the processor could control the heater to stop heating, thereby dry burning is avoided when the electronic atomizing apparatus is tilted, and the components of the electronic atomizing apparatus is effectively protected from damage, harmful substances are prevented from being produced due to the dry burning of the aerosol-generating liquid, and the harm to the user's health is reduced.

### BRIEF DESCRIPTION OF THE DRAWINGS

In order to more clearly illustrate the technical solution in embodiments of the present disclosure, the drawings used in the description of the embodiments will be briefly introduced below. Obviously, the drawings in the following description are just some embodiments of the present disclosure. For those of ordinary skill in the art, other drawings could be obtained according to these drawings without paying creative works.
Fig. 1 is a sectional view of an electronic atomizing apparatus according to an embodiment of the present disclosure.
Fig. 2 is a schematic structural diagram of an electronic atomizing apparatus according to another embodiment of the present disclosure.
Fig. 3 is a schematic diagram of an electronic atomizing apparatus of the present disclosure in a tilted state.
Fig. 4 is a sectional view of an atomizer used in an electronic atomizing apparatus according to an embodiment of the present disclosure.
Fig. 5 is a sectional view of a power supply assembly used in an electronic atomizing apparatus according to an embodiment of the present disclosure.
Fig. 6 is a schematic flowchart of a control method of an electronic atomizing apparatus according to an embodiment of the present disclosure.

### DETAILED DESCRIPTION

The technical solution in embodiments of the present disclosure will be clearly and thoroughly described with reference to the drawings in embodiments of the present disclosure. Obviously, the described embodiments are merely part of the embodiments of the present disclosure, but not all of them. Based on the embodiments in the present disclosure, all other embodiments obtained by a person of ordinary skill in the art without creative works shall fall within the protection scope of the present disclosure.

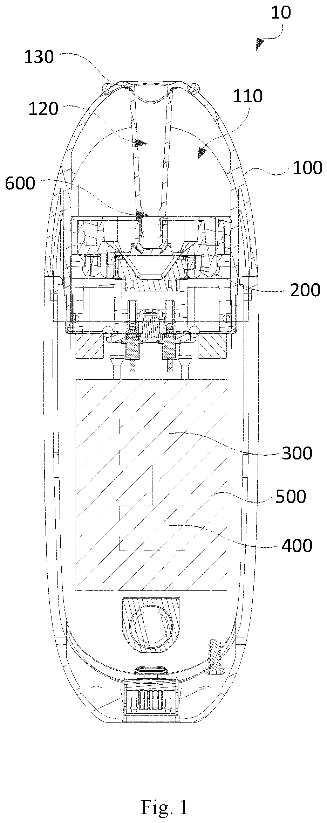

Referring to Fig. 1, an electronic atomizing apparatus 10 according to one embodiment of the present disclosure includes a housing 100, a heater 200, an angle detection mechanism 300, a processor 400 and a battery 500. The housing 100 is configured to define a liquid storage chamber 110, an air flow channel 120 and a mouthpiece 130. The liquid storage chamber 110 communicates with the airflow channel 120 through the heater 200. The airflow channel 120 communicates with the atmosphere through the mouthpiece 130. The heater 200, each of the angle detection mechanism 300, the processor 400 and the battery 500 is arranged in the housing 100. The liquid storage chamber 110 is arranged between the mouthpiece 130 and the heater 200 and could be configured to storage the aerosol-generating liquid. The processor 400 is connected to the heater 200 and the angle detection mechanism 300. The heater 200 is configured to atomize the aerosol-generating liquid (not shown in the figure) in the liquid storage chamber 110 by heating. The battery 500 is connected to the heater 200, and is configured to supply power to the heater 200 for atomizing by heating. The angle detection mechanism 300 is configured to detect the tilt angle of the electronic atomizing apparatus 10. The processor 400 is configured to determine whether the tilt angle is less than an angle threshold. When it is determined that the tilt angle is less than the angle threshold, the processor 400 would control the heater 200 to start heating. When it is determined that the tilt angle is greater or equal to the angle threshold, the processor 400 would control the heater 200 to stop heating.

In the embodiments of the present disclosure, the angle detection mechanism is arranged in the electronic atomizing apparatus to detect the tilt angle of the electronic atomizing apparatus. When it is determined that the tilt angle is less than the angle threshold, the processor could control the heater to stop heating, thereby avoiding dry burning when the electronic atomizing apparatus is tilted, and preventing the components of the electronic atomizing apparatus from being damaged. Moreover, production of harmful substances could be prevented due to the dry burning of the aerosol-generating liquid, and thereby reducing harm to the health of the user.

In the present embodiment, the angle detection mechanism 300 and the processor 400 are both arranged on the battery 500. In other embodiments, the angle detection mechanism is also directly arranged in other locations of the housing 100. The angle detection mechanism is arranged in a location adjacent to the heater 200, or at a side of the battery 500 away from the heater 200, which is not limited herein.

In the present embodiment, the angle detection mechanism 300 is an angle sensor configured to directly detect the tilt angle of the electronic atomizing apparatus 10, specifically a gravity sensor or a tilt sensor. The tilt angle of the electronic atomizing apparatus 10 could be detected by the angle sensor more accurately, and the angle sensor occupies a small space, which could facilitate the miniaturization of the electronic atomizing apparatus 10.

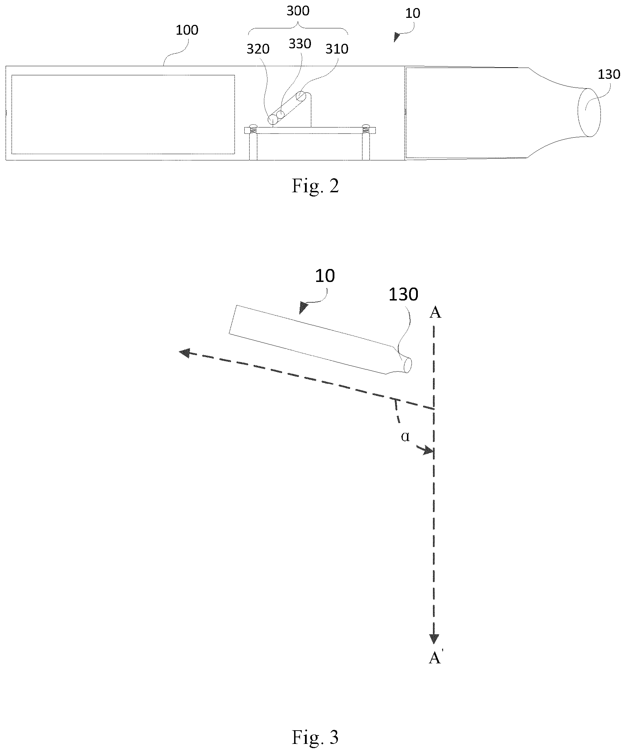

Referring to Fig. 2 together, in another embodiment, the angle detection mechanism 300 is a ball switch. The ball switch includes a conducting terminal 310, a disconnecting terminal 320, and a ball 330 arranged between the conducting terminal 310 and the disconnecting terminal 320. The ball 330 is configured to abut the conducting terminal 310 and the disconnecting terminal 320 respectively.

In the present embodiment, when the tilt angle of the electronic atomizing apparatus 10 is less than the angle threshold, the ball 330 would roll to abut the disconnecting terminal 320. When the tilt angle of the electronic atomizing apparatus 10 is greater or equal to the angle threshold, the ball 330 would roll to abut the conducting terminal 310. The processor 400 is also configured to detect the abutment between the ball 330 and the conducting terminal 310 or the abutment between the ball 330 and the disconnecting terminal 320. If the abutment between the ball 330 and the disconnecting terminal 320 is detected, the processor 400 would control the heater 200 to start heating; and if the abutment between the ball 330 and the conducting terminal 310 is detected, the processor 400 would control the heater 200 to stop heating.

In the present embodiment, the electronic atomizing apparatus 10 further includes an alerter (not shown in the figures). The alerter is communicatively connected to the processor 400, and is configured to alert the user of wrong operation manner of the electronic atomizing apparatus 10 when the tilt angle of the electronic atomizing apparatus 10 is greater or equal to the angle threshold. The alerter is an alerting light or an audio alert and so on.

Referring also to Fig. 3, the tilt angle α is the angle between the opposite direction of orientation of the mouthpiece 130 of the electronic atomizing apparatus 10 and the vertical downward direction AA', and the angle threshold could be from 90° to 150°, such as 90°, 120°, or 150°.

In other embodiments, multiple angle thresholds is also set, such as a first angle threshold and a second angle threshold are set. The first angle threshold is less than the second angle threshold. When the tilt angle of the electronic atomizing apparatus 10 is greater or equal to the first angle threshold and less than the second angle threshold, the processor 400 would control the heater 200 to reduce its heating power. When the tilt angle of the electronic atomizing apparatus 10 is greater or equal to the angle threshold, the processor 400 would control the heater 200 to stop heating. The first angle threshold is from 90° to 120°, such as 90°, 100°, or 120°; the second angle threshold is from 120° to 150°, such as 120°, 140°, or 150°.

In other embodiments, three or more than three angle thresholds could be set, and multiple heating power stages could be correspondingly set.

In the present embodiment, multiple angle thresholds are set, thus the electronic atomizing apparatus 10 could have different heating powers at different tilt angle, so that when the electronic atomizing apparatus 10 is tilted, overheating due to the amount of the aerosol-generating liquid heated by a part of the heater being too low could thereby be avoided.

In the present embodiment, the electronic atomizing apparatus 10 further includes a trigger switch (not shown in the figures). The trigger switch is communicatively connected to the processor 400. The trigger switch is configured to receive a trigger instruction and send the trigger instruction to the processor 400. The processor 400 controls the heater 200 to start heating upon receiving the trigger instruction and receives the tilt angle information detected by the angle detection mechanism 300.

In the present embodiment, a choke switch 600 is further arranged in the airflow channel 120. The choke switch 600 is configured to open the airflow channel 120 when the tilt angle is less than the angle threshold, and close the airflow channel 120 when the tilt angle is greater than or equal to the angle threshold.

In other embodiments, the choke switch is also arranged in the liquid storage chamber 110, which is not limited herein.

In the present embodiment, the tilt angle of the electronic atomizing apparatus is detected by the angle sensor or the ball switch, and the accuracy is higher. By arranging an alerter, the user could be timely warned to correct the operation manner of the electronic atomizing apparatus, thereby prolonging the service life of the electronic atomizing apparatus. By arranging the angle detection mechanism and the processor on a battery with larger cross sectional area, the structure of the electronic atomizing apparatus could be more stable and the inner space of the electronic atomizing apparatus could be more fully used, and the structure could be more compact. By arranging a choke switch, leakage of the aerosol-generating liquid from the mouthpiece when the electronic atomizing apparatus is inverted could be avoided, thus avoiding the waste of the aerosol-generating liquid and the contamination of the environment.

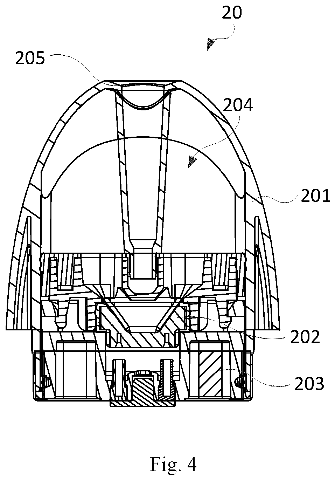

Referring to Fig. 4, an atomizer 20 configured to be used in an electronic atomizing apparatus according to an embodiment of the present disclosure includes an atomizer housing 201, a heater 202 and an angle detection mechanism 203. The atomizer housing 201 is configured to define a liquid storage chamber 204 and a mouthpiece 205. The liquid storage chamber 204 is configured to storage aerosol-generating liquid. The heater 202 is arranged in the atomizer housing 201, powered by a power supply assembly (not shown in the figures) of the electronic atomizing apparatus, and configured to atomize the aerosol-generating liquid by heating. The liquid storage chamber 204 is arranged between the mouthpiece 205 and the heater 202. The angle detection mechanism 203 is arranged in the atomizer housing 201 and is configured to detect the tilt angle of the electronic atomizing apparatus. The heater 202 and the angle detection mechanism 203 is also configured to connect with the processor of the electronic atomizing apparatus, such that the processor could determine whether the tilt angle is less than the angle threshold. When it is determined that the tilt angle is less than the angle threshold, the processor would control the heater 202 to start heating. When it is determined that the tilt angle is greater or equal to the angle threshold, the processor could control the heater 202 to stop heating.

In the present embodiment, the angle detection mechanism 203 is an angle sensor or a ball switch. Specifically, referring to the foregoing embodiments of the electronic atomizing apparatus 10, the details are not described herein again.

In the embodiments of the present disclosure, an angle detection mechanism is arranged on the atomizer of the electronic atomizing apparatus to detect the tilt angle of the electronic atomizing apparatus. When it is determined that the tilt angle is less than the angle threshold, the processor of the electronic atomizing apparatus could control the heater to stop heating. Thereby dry burning could be avoided when the electronic atomizing apparatus is tilted, and the components of the electronic atomizing apparatus could be effectively protected from damage, harmful substances could be prevented from being produced due to the dry burning of the aerosol-generating liquid, and the harm to the user's health could be reduced.

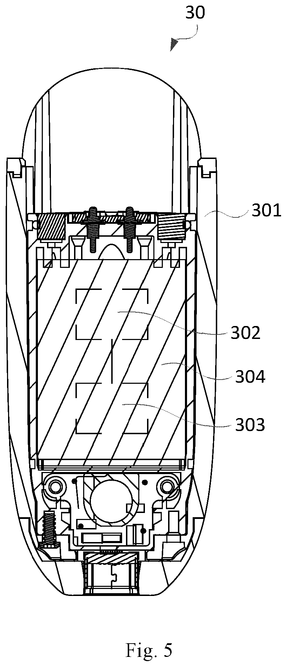

Referring to Fig. 5, the power supply assembly 30 configured to be used in the electronic atomizing apparatus according to one embodiment of the present disclosure includes a power supply assembly housing 301, a processor 303 and a battery 304 arranged in the power supply assembly housing 301. A heater (not shown in the figures) and an angle detection mechanism 302 is arranged in the electronic atomizing apparatus. The heater, the battery 304 and the angle detection mechanism 302 are all connected to the processor 303. The angle detection mechanism 302 is configured to detect the tilt angle of the electronic atomizing apparatus. The processor 303 is configured to determine whether the tilt angle is less than the angle threshold. When it is determined that the tilt angle is less than the angle threshold, the processor 303 would control the battery 304 so that the heater could start heating. When it is determined that the tilt angle is greater or equal to the angle threshold, the processor 303 would control the battery 304 so that the heater could stop heating.

In the present embodiment, the angle detection mechanism 302 is arranged in the power supply assembly housing 301. In other embodiments, the angle detection mechanism could also be arranged in the atomizer of the electronic atomizing apparatus. When the power supply assembly 30 and the atomizer are assembled to create an electronic atomizing apparatus, the processor 303 of the power supply assembly 30 and the angle detection mechanism of the atomizer could be coupled to control the heater depending on the tilt angle detected by the angle detection mechanism.

In the present embodiment, the angle detection mechanism 302 is an angle sensor or a ball switch. Specifically, referring to the foregoing embodiments of the electronic atomizing apparatus 10, the details are not described herein again.

In the embodiments of the present disclosure, an angle detection mechanism is arranged on the power supply assembly of the electronic atomizing apparatus to detect the tilt angle of the electronic atomizing apparatus. When it is determined that the tilt angle is less than the angle threshold, the processor could control the heater to stop heating, thereby dry burning is avoided when the electronic atomizing apparatus is tilted, and the components of the electronic atomizing apparatus is effectively protected from damage, harmful substances are prevented from being produced due to the dry burning of the aerosol-generating liquid, and the harm to the user's health is reduced.

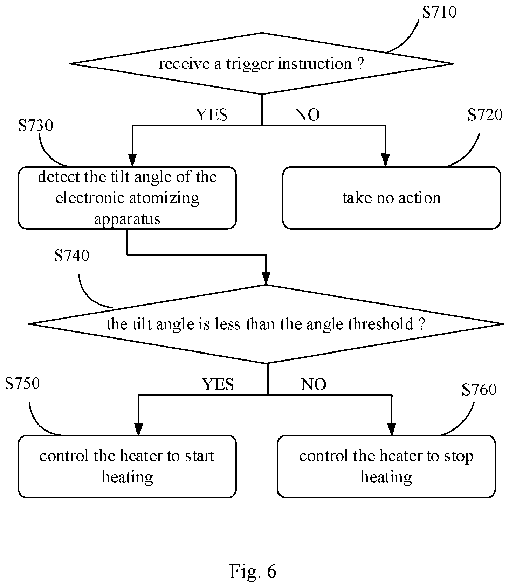

Referring to Fig. 6, a control method of the electronic atomizing apparatus according to an embodiment of the present disclosure includes the following operations.

Operation S710, a determination is made about whether a trigger instruction is received.

In the present embodiment, the trigger instruction could be triggered by depressing a button or by voice command and so on.

Operation S720, when no trigger instruction is received, then no action is taken.

Operation S730, when a trigger instruction is received, then the tilt angle of the electronic atomizing apparatus is detected.

In the present embodiment, the tilt angle of the electronic atomizing apparatus is detected by an angle detection mechanism. The angle detection mechanism could be an angle sensor or a ball switch and so on.

Operation S740, a determination is made about whether the tilt angle is less than the angle threshold.

In the present embodiment, the tilt angle is the angle between the opposite direction of the orientation of the mouthpiece of the electronic atomizing apparatus and the vertical downward direction, and the angle threshold is from 90° to 150°.

Operation S750, when the tilt angle is less than the angle threshold, then the heater is controlled to start heating.

Operation S760, when the tilt angle is greater or equal to the angle threshold, then the heater is controlled to stop heating.

Specifically, for the structure of the electronic atomizing apparatus for implementing the control method, please refer to the foregoing embodiments of electronic atomizing apparatus, the details are not described herein again.

In the embodiments of the present disclosure, an angle detection mechanism is arranged in the electronic atomizing apparatus to detect the tilt angle of the electronic atomizing apparatus. When it is determined that the tilt angle is less than the angle threshold, the processor could control the heater to stop heating, thereby dry burning could be avoided when the electronic atomizing apparatus is tilted, and the components of the electronic atomizing apparatus could be effectively protected from damage, harmful substances could be prevented from being produced due to the dry burning of the aerosol-generating liquid, and the harm to the user's health could be reduced.

## Claims

1. An electronic atomizing apparatus (10), **characterized by** comprising:
a housing (100), configured to define a liquid storage chamber (110) and a mouthpiece (130), the liquid storage chamber (110) is configured to storage aerosol-generating liquid;
a heater (200), arranged in the housing (100) and configured to atomize the aerosol-generating liquid by heating, the liquid storage chamber (110) is arranged between the mouthpiece (130) and the heater (200);
a battery (500, 304), arranged in the housing (100) and connected to the heater (200), and configured to supply power to the heater (200) to atomize by heating;
an angle detection mechanism (300, 203, 302), arranged in the housing (100), and configured to detect a tilt angle (α) of the electronic atomizing apparatus (10);
a processor (400, 303), arranged in the housing (100) and connected to the heater (200) and the angle detection mechanism (300, 203, 302), the processor (400, 303) is configured to determine whether the tilt angle (α) is less than the angle threshold, when it is determined that the tilt angle (α) is less than the angle threshold, the processor (400, 303) controls the heater (200) to start heating; when it is determined that the tilt angle (α) is greater or equal to the angle threshold, the processor (400, 303) controls the heater (200) to stop heating,
wherein the housing (100) further defines an airflow channel (120), the liquid storage chamber (110) communicates with the airflow channel (120) through the heater (200), the airflow channel (120) communicates with the atmosphere through the mouthpiece (130), a choke switch (600) is arranged in the airflow channel (120) or the liquid storage chamber (110), the choke switch (600) is configured to open the airflow channel (120) when the tilt angle (α) is less than the angle threshold, and close the airflow channel (120) when the tilt angle (α) is greater or equal to the angle threshold.

2. The electronic atomizing apparatus (10) according to claim 1, **characterized in that** the angle detection mechanism (300, 203, 302) is an angle sensor.

3. The electronic atomizing apparatus (10) according to claim 1, **characterized in that** the angle detection mechanism (300, 203, 302) is a ball switch, the ball switch comprises a conducting terminal (310), a disconnecting terminal (320) and a ball (330) arranged between the conducting terminal (310) and the disconnecting terminal (320), the ball (330) is configured to abut the conducting terminal (310) and the disconnecting terminal (320) respectively.

4. The electronic atomizing apparatus (10) according to claim 3, **characterized in that** when the tilt angle (α) of the electronic atomizing apparatus (10) is less than the angle threshold, the ball (330) rolls to abut the disconnecting terminal (320); when the tilt angle (α) of the electronic atomizing apparatus (10) is greater or equal to the angle threshold, the ball (330) rolls to abut the conducting terminal (310).

5. The electronic atomizing apparatus (10) according to claim 3 or 4, **characterized in that** the processor (400, 303) is further configured to detect the abutment between the ball (330) and the conducting terminal (310), and detect the abutment between the ball (330) and the disconnecting terminal (320), when the abutment between the ball (330) and the disconnecting terminal (320) is detected, the processor (400, 303) controls the heater (200) to start heating; when the abutment between the ball (330) and the conducting terminal (310) is detected, the processor (400, 303) controls the heater (200) to stop heating.

6. The electronic atomizing apparatus (10) according to any one of claims 1 to 5, **characterized in that** the tilt angle (α) is the angle between the opposite direction of the orientation of the mouthpiece (130) of the electronic atomizing apparatus (10) and the vertical downward direction (AA'), the angle threshold is from 90° to 150°.

7. The electronic atomizing apparatus (10) according to any one of claims 1 to 6, **characterized in that** the electronic atomizing apparatus (10) further comprises a trigger switch, the trigger switch is connected to the processor (400, 303), the trigger switch is configured to receive a trigger instruction and send the trigger instruction to the processor (400, 303).

8. The electronic atomizing apparatus (10) according to claim 1, **characterized by** comprising an atomizer (20), the atomizer (20) comprises an atomizer housing (201);
the angle detection mechanism (203) is arranged in the atomizer housing (201).

9. The electronic atomizing apparatus (10) according to claim 1, **characterized by** comprising a power supply assembly (30), the power supply assembly (30) comprises a power supply assembly housing (301), the battery (304) and the processor (303) are arranged in the power supply assembly housing (301), wherein when it is determined that the tilt angle (α) is less than the angle threshold, the processor (303) controls the battery (304) to make the heater (200) to start heating; when it is determined that the tilt angle (α) is greater or equal to the angle threshold, the processor (303) controls the battery (304) to make the heater (200) to stop heating.

10. The electronic atomizing apparatus (10) according to claim 9, **characterized in that** the angle detection mechanism (302) is arranged in the power supply assembly housing (301).

11. A control method of the electronic atomizing apparatus (10) as claimed in any of claims 1-10, **characterized by** comprising:
detecting (S730) a tilt angle (α) of the electronic atomizing apparatus (10);
determining (S740) whether the tilt angle (α) is less than an angle threshold;
controlling (S750) a heater (200) to start heating when the tilt angle (α) is less than the angle threshold;
controlling (S760) the heater (200) to stop heating when the tilt angle (α) is greater or equal to the angle threshold;
controlling a choke switch (600) to open an airflow channel (120) when the tilt angle (α) is less than the angle threshold;
controlling the choke switch (600) to close the airflow channel (120) when the tilt angle (α) is greater or equal to the angle threshold.

12. The control method according to claim 11, wherein before the detecting a tilt angle (α) of the electronic atomizing apparatus (10), the control method further comprises:
determining (S710) whether a trigger instruction is received;
detecting (S730) the tilt angle (α) of the electronic atomizing apparatus (10) if a trigger instruction is received;
taking (S720) no actions if no trigger instruction is received.

13. The control method according to claims 11 or 12, wherein the tilt angle (α) of the electronic atomizing apparatus (10) is detected by an angle detection mechanism (300, 203, 302), the angle detection mechanism (300, 203, 302) is an angle sensor or a ball switch.

## Patentansprüche

1. Ein elektronischer Zerstäubungsapparat (10) **dadurch gekennzeichnet, dass** er Folgendes umfasst:
Ein Gehäuse (100), das für die Definierung einer Speicherkammer (110) für Flüssigkeiten und eines Mundstücks (130) konfiguriert ist, wobei die Speicherkammer (110) für Flüssigkeiten konfiguriert ist, um eine ein Aerosol erzeugende Flüssigkeit zu speichern;
Ein Heizkörper (200), der im Gehäuse angeordnet und konfiguriert ist, um die das Aerosol erzeugende Flüssigkeit durch Erhitzen zu zerstäuben, wobei die Speicherkammer (110) für Flüssigkeiten zwischen dem Mundstück (130) und
dem Heizkörper (200) angeordnet ist;
Eine Batterie (500, 304), die im Gehäuse (100) angeordnet und an den Heizkörper (200) angeschlossen ist, und konfiguriert ist, um den Heizkörper (200) zum Zerstäuben durch Erhitzen mit Energie zu versorgen;
Ein im Gehäuse (100) angeordneter Mechanismus (300, 203, 302) zur Feststellung eines Winkels, Mechanismus, der zur Feststellung eines Neigungswinkels (α) des elektronischen Zerstäubungsapparats (10) konfiguriert ist;
Einen im Gehäuse (100) angeordneten Prozessor (400, 303), der an den Heizkörper (200) und an den Mechanismus (300, 303, 302) zur Feststellung eines Winkels angeschlossen ist, wobei der Prozessor (400, 303) konfiguriert ist, um zu bestimmen, ob der Neigungswinkel (α) kleiner ist als der Winkelgrenzwert, wenn festgestellt wird, dass der Neigungswinkel (α) kleiner ist als der Winkelgrenzwert, steuert der Prozessor (400, 303) den Heizkörper (200), damit dieser mit dem Erhitzen beginnt; wenn festgestellt wird, dass der Neigungswinkel (α) grösser ist als der oder gleich dem Winkelgrenzwert, steuert der Prozessor (400, 303) den Heizkörper (200) damit dieser das Erhitzen abbricht,
wobei das Gehäuse (100) weiter einen Luftstromkanal (120) definiert, die Speicherkammer (110) für Flüssigkeiten mit dem Luftstromkanal (120) durch den Heizkörper (200) kommuniziert, der Luftstromkanal (120) durch das Mundstück (130) mit der Umgebung kommuniziert, ein Drosselschalter (600) im Luftstromkanal (120) oder der Speicherkammer (110) für Flüssigkeiten angeordnet ist, der Drosselschalter (600) zum Öffnen des Luftstromkanals (120) konfiguriert ist, wenn der Neigungswinkel (α) kleiner ist als der Winkelgrenzwert, und zum Schliessen des Luftstromkanals (120) wenn der Neigungswinkel (α) grösser ist als der oder gleich des Winkelgrenzwertes.

2. Der elektronische Zerstäubungsapparat (10) gemäss Anspruch 1, **dadurch gekennzeichnet, dass** der Mechanismus (300, 203, 302) für die Feststellung des Winkels ein Winkelsensor ist.

3. Der elektronische Zerstäubungsapparat (10) gemäss Anspruch 1, **dadurch gekennzeichnet, dass** der Mechanismus (300, 203, 302) für die Feststellung des Winkels ein Kugelschalter ist, der Kugelschalter eine leitende Anschlussklemme (310), ein Abschaltterminal (320) und eine Kugel (330) umfasst, die zwischen der leitenden Anschlussklemme (310) und dem Abschaltterminal (320) angeordnet ist, wobei die Kugel (330) jeweils zum Angrenzen an das leitende Terminal (310) und das Abschaltterminal (320) konfiguriert ist.

4. Der elektronische Zerstäubungsapparat (10) gemäss Anspruch 3, **dadurch gekennzeichnet, dass** der Neigungswinkel (α) des elektronischen Zerstäubungsapparats (10) kleiner ist als der Winkelgrenzwert, wobei die Kugel (330) rollt, um an das Abschaltterminal (320) zu stossen; wenn der Neigungswinkel (α) des elektronischen Zerstäubungsapparats (10) grösser ist als der oder gleich dem Winkelgrenzwert, rollt die Kugel, um an die leitendende Anschlussklemme (310) anzuschlagen.

5. Der elektronische Zerstäubungsapparat (10) gemäss Anspruch 3 oder 4, **dadurch gekennzeichnet, dass** der Prozessor (400, 303) weiter konfiguriert ist, um den Anschlag zwischen der Kugel (330) und der leitenden Anschlussklemme (310) festzustellen, und stellt den Anschlag zwischen der Kugel (330) und dem Abschaltterminal (320) fest; wenn der Anschlag zwischen der Kugel (330) und dem Abschaltterminal (320) erkannt wird, steuert der Prozessor (400, 303) den Heizkörper (200), um den Erwärmungsvorgang einzuleiten; wenn der Anschlag zwischen der Kugel (330) und der leitenden Anschlussklemme (310) erkannt wird, steuert der Prozessor (400, 303) den Heizkörper, um den Erwärmungsvorgang abzubrechen.

6. Der elektronische Zerstäubungsapparat (10) gemäss irgendeinem der Ansprüche 1 bis 5, **dadurch gekennzeichnet, dass** der Neigungswinkel (α) der Winkel zwischen gegenüberliegenden Orientierungsrichtungen des Mundstücks (130) des elektronischen Zerstäubungsapparats (10) und der vertikalen Richtung (A, A') nach unten ist, wobei der Winkelgrenzwert von 90º bis 150º beträgt.

7. Der elektronische Zerstäubungsapparat (10) gemäss irgendeinem der Ansprüche 1 bis 6, **dadurch gekennzeichnet, dass** der elektronische Zerstäubungsapparat (10) weiter einen Auslöseschalter umfasst, wobei der Auslöseschalter an den Prozessor (400, 303) angeschlossen und konfiguriert ist, um eine Auslöseanweisung zu empfangen und die Auslöseanweisung an den Prozessor (400, 303) weiterzuleiten.

8. Der elektronische Zerstäubungsapparat (10) gemäss Anspruch 1, **dadurch gekennzeichnet, dass** er einen Zerstäuber (20) umfasst, wobei der Zerstäuber (20) ein Zerstäubergehäuse (201) umfasst, der Mechanismus (203) zur Feststellung des Winkels in dem Zerstäubergehäuse (201) angeordnet ist.

9. Der elektronische Zerstäubungsapparat (10) gemäss Anspruch 1, **dadurch gekennzeichnet, dass** er eine Stromversorgungsgruppe (30) umfasst, die Stromversorgungsgruppe (30) ein Gehäuse (301) für die Stromversorgungsgruppe (30) umfasst, die Batterie (304) und der Prozessor (303) im Gehäuse (301) für die Stromversorgungsgruppe (30) angeordnet sind, wobei, wenn festgestellt wird, dass der Neigungswinkel (α) geringer ist als der Winkelgrenzwert, der Prozessor die Batterie (304) so steuert, dass der Heizkörper (200) mit dem Erhitzen beginnt; wenn festgestellt wird, dass der Neigungswinkel (α) grösser ist als der oder gleich dem Winkelgrenzwert, steuert der Prozessor (303) die Batterie (304) und bringt den Heizkörper (200) zum Anhalten des Erhitzungsvorgangs.

10. Der elektronische Zerstäubungsapparat (10) gemäss Anspruch 9, **dadurch gekennzeichnet, dass** der Mechanismus (302) für die Feststellung des Winkels im Gehäuse (301) der Stromversorgungsgruppe angeordnet ist.

11. Ein Steuerverfahren für den elektronischen Zerstäubungsapparat (10) gemäss irgendeinem der Ansprüche 1-10, **dadurch gekennzeichnet, dass** es Folgendes umfasst:
Erfassung (S730) eines Neigungswinkels (α) des elektronischen Zerstäubungsapparats (10);
Bestimmung (S740), ob der Neigungswinkel (α) geringer ist als ein Winkelgrenzwert;
Steuerung (S750) eines Heizkörpers (200) zwecks Starten des Erhitzungsvorganges, wenn der Neigungswinkel (α) geringer ist als der Winkelgrenzwert;
Steuerung (S760) des Heizkörpers (200) zwecks Anhalten des Erhitzungsvorgangs, wenn der Neigungswinkel (α) grösser ist als der oder gleich dem Winkelgrenzwert;
Steuerung des Drosselschalters (600) zum Öffnen eines Luftstromkanals (120), wenn der Neigungswinkel (α) geringer ist als der Winkelgrenzwert;
Steuerung des Drosselschalters (600) zum Schliessen des Luftstromkanals (120), wenn der Neigungwinkel (α) grösser ist als der oder gleich dem Winkelgrenzwert.

12. Das Steuerverfahren gemäss Anspruch 11, bei dem vor der Erfassung eines Neigungswinkels (α) des elektronischen Zerstäubungsapparats (10) das Steuerverfahren weiter Folgendes umfasst:
Bestimmung (S710), ob eine Auslöseanweisung empfangen wurde;
Erfassung (S730) des Neigungswinkels (α) des elektronischen Zerstäubungsapparats (10), wenn eine Auslöseanweisung empfangen wurde;
(S720) funktioniert nicht, wenn keine Auslöseanweisung eingetroffen ist.

13. Das Steuerverfahren gemäss Anspruch 11 oder 12, bei dem der Neigungswinkel (α) des elektronischen Zerstäubungsapparats (10) durch einen Mechanismus (300, 203, 302) zur Feststellung eines Winkels erfasst wird, wobei der Mechanismus (300, 203, 302) zur Feststellung eines Winkels ein Winkelsensor oder ein Kugelschalter ist.

## Revendications

1. Appareil électronique de pulvérisation (10), **caractérisé en ce qu'**il comprend:
un boîtier (100), configuré pour définir une chambre de stockage de liquide (110) et un embout (130), la chambre de stockage de liquide (110) étant configurée pour stocker le liquide générant l'aérosol;
un dispositif de chauffage (200), disposé dans le boîtier (100) et configuré pour atomiser le liquide générateur d'aérosol par chauffage, la chambre de stockage de liquide (110) étant disposée entre l'embout (130) et le dispositif de chauffage (200);
une batterie (500, 304), disposée dans le boîtier (100) et connectée à l'élément chauffant (200), et configurée pour alimenter l'élément chauffant (200) afin d'atomiser par chauffage;
un mécanisme de détection d'angle (300, 203, 302), disposée dans le boîtier (100) et configuré pour détecter un angle d'inclinaison (α) de l'appareil électronique de pulvérisation (10);
un processeur (400, 303), disposée dans le boîtier (100) et connecté au dispositif de chauffage (200) et au mécanisme de détection d'angle (300, 203, 302), le processeur (400, 303) étant configuré pour déterminer si l'angle d'inclinaison (α) est inférieur à la valeur limite angulaire, et lorsque a été déterminé que l'angle d'inclinaison (α) est inférieur à la valeur limite angulaire, le processeur (400, 303) commande le dispositif de chauffage (200) pour qu'il commence à chauffer; lorsqu'il est déterminé que l'angle d'inclinaison (α) est supérieur ou égal à la valeur limite angulaire, le processeur (400, 303) commande le dispositif de chauffage (200) pour arrêter le chauffage,
où le boîtier (100) définit en outre un canal de circulation d'air (120), la chambre de stockage de liquide (110) étant en communication avec le canal de circulation d'air (120) par l'intermédiaire de l'élément chauffant (200), le canal de circulation d'air (120) étant en communication avec l'environnement par l'intermédiaire de l'embout buccal (130), un commutateur d'étranglement (600) est disposé dans le canal de circulation d'air (120) ou dans la chambre de stockage de liquide (110), le commutateur d'étranglement (600) étant configuré pour ouvrir le canal de circulation d'air (120) lorsque l'angle d'inclinaison (α) est inférieur à la valeur limite angulaire, et pour fermer le canal de circulation d'air (120) lorsque l'angle d'inclinaison (α) est supérieur ou à la valeur limite angulaire.

2. Appareil électronique de pulvérisation (10) selon la revendication 1, **caractérisé en ce que** le mécanisme de détection d'angle (300, 203, 302) est un capteur d'angle.

3. Appareil électronique de pulvérisation (10) selon la revendication 1, **caractérisé en ce que** le mécanisme de détection d'angle (300, 203, 302) est un commutateur à bille, le commutateur à bille comprenant une borne conductrice (310), une borne de déconnexion (320) et une bille (330) disposée entre la borne conductrice (310) et la borne de déconnexion (320), la bille (330) étant configurée pour venir en butée contre la borne conductrice (310) et la borne de déconnexion (320), respectivement.

4. Appareil électronique de pulvérisation (10) selon la revendication 3, **caractérisé en ce que** lorsque l'angle d'inclinaison (α) de l'appareil électronique de pulvérisation (10) est inférieur à la valeur limite angulaire, la bille (330) roule pour venir en butée contre la borne de déconnexion (320); lorsque l'angle d'inclinaison (α) de l'appareil électronique de pulvérisation (10) est supérieur ou égal à la valeur limite angulaire, la bille (330) roule pour venir en butée contre la borne conductrice (310).

5. Appareil électronique de pulvérisation (10) selon la revendication 3 ou 4, **caractérisé en ce que** le processeur (400, 303) est en outre configuré pour détecter le contact entre la bille (330) et la borne conductrice (310), et détecter le contact entre la bille (330) et la borne de déconnexion (320), et lorsque le contact entre la bille (330) et la borne de déconnexion (320) est détecté, le processeur (400, 303) commande l'appareil de chauffage (200) pour initier l'opération de chauffage; lorsque le contact entre la bille (330) et la borne conductrice (310) est détecté, le processeur (400, 303) commande l'arrêt du chauffage (200).

6. Appareil électronique de pulvérisation (10) selon l'une quelconque des revendications 1 à 5, **caractérisé en ce que** l'angle d'inclinaison (α) est l'angle entre la direction opposée de l'orientation de l'embout buccal (130) de l'appareil électronique de pulvérisation (10) et la direction verticale vers le bas (AA'), la valeur limite angulaire étant comprise entre 90º et 150º.

7. Appareil électronique de pulvérisation (10) selon l'une quelconque des revendications 1 à 6, **caractérisé en ce que** l'appareil électronique de pulvérisation (10) comprend en outre un interrupteur de déclenchement, l'interrupteur de déclenchement étant connecté au processeur (400, 303), et l'interrupteur de déclenchement étant configuré pour recevoir une instruction de déclenchement et pour transmettre l'instruction de déclenchement au processeur (400, 303).

8. Appareil électronique de pulvérisation (10) selon la revendication 1, **caractérisé par le fait qu'**il comprend un atomiseur (20), l'atomiseur (20) comprenant un boîtier d'atomiseur (201); dans lequel le mécanisme de détection d'angle (203) est disposé dans le boîtier d'atomiseur (201).

9. Appareil électronique de pulvérisation (10) selon la revendication 1, **caractérisé en ce qu'**il comprend un dispositif d'alimentation électrique (30), le dispositif d'alimentation électrique (30) comprenant un boîtier pour le dispositif d'alimentation électrique (301), la batterie (304) et le processeur (303) étant disposés dans le boîtier du dispositif d'alimentation électrique (301), dans lequel, lorsqu'il est déterminé que l'angle d'inclinaison (α) est inférieur à la limite angulaire, le processeur (303) commande la batterie (304) de sorte que l'élément chauffant (200) commence à chauffer; lorsqu'il est déterminé que l'angle d'inclinaison (α) est supérieur ou égal á la valeur limite angulaire, le processeur (303) commande la batterie (304) et amène le dispositif de chauffage (200) à arrêter le processus de chauffage.

10. Appareil électronique de pulvérisation (10) selon la revendication 9, **caractérisé en ce que** le mécanisme de détection d'angle (302) est disposé dans le boîtier du dispositif d'alimentation électrique (301).

11. Procédé de contrôle de l'appareil électronique de pulvérisation (10) selon l'une quelconque des revendications 1 à 10, **caractérisé en ce qu'**il comprend les étapes suivantes:
détecter (S730) un angle d'inclinaison (α) de l'appareil électronique de pulvérisation (10);
déterminer (S740) si l'angle d'inclinaison (α) est inférieur à une valeur limite angulaire spécifique;
commander (S750) un dispositif de chauffage (200) pour commencer à chauffer lorsque l'angle d'inclinaison (α) est inférieur à la valeur limite angulaire;
commander (S760) le dispositif de chauffage (200) pour arrêter le processus de chauffage lorsque l'angle d'inclinaison (α) est supérieur ou égal au à la valeur limite angulaire;
commander un commutateur d'étranglement (600) pour ouvrir un canal de circulation d'air (120) lorsque l'angle d'inclinaison (α) est inférieur à la valeur limite angulaire;
commander le commutateur d'étranglement (600) pour fermer le canal de circulation d'air (120) lorsque l'angle d'inclinaison (α) est supérieur ou égal à la valeur limite angulaire.

12. Procédé de contrôle selon la revendication 11, dans lequel avant la détection d'un angle d'inclinaison (α) de l'appareil électronique d'atomisation (10), le procédé de contrôle comprend en outre les étapes suivantes:
déterminer (S710) si une instruction de déclenchement a été reçue ou non;
détecter (S730) l'angle d'inclinaison (α) de l'appareil électronique de pulvérisation (10) si une instruction de déclenchement a été reçue;
ne prendre (S720) aucune mesure lorsqu'aucune instruction de déclenchement n'a été reçue.

13. Procédé de commande selon les revendications 11 ou 12, dans lequel l'angle d'inclinaison (α) de l'appareil électronique de pulvérisation (10) est détecté par un mécanisme de détection d'angle (300, 203, 302), le mécanisme de détection d'angle (300, 203, 302) étant un capteur d'angle ou un commutateur à bille.
